Europäisches Patentamt

European Patent Office

Office européen des brevets

(19)

(11) **EP 1 213 027 A2**

(12) **EUROPEAN PATENT APPLICATION**

(43) Date of publication:
**12.06.2002 Bulletin 2002/24**

(51) Int Cl.7: **A61K 35/78**, A23L 1/00,
A23L 2/00, A61P 1/16

(21) Application number: **01129254.7**

(22) Date of filing: **11.12.2001**

(84) Designated Contracting States:
**AT BE CH CY DE DK ES FI FR GB GR IE IT LI LU
MC NL PT SE TR**
Designated Extension States:
**AL LT LV MK RO SI**

(30) Priority: **11.12.2000 JP 2000375510**

(71) Applicant: **KYOWA HAKKO KOGYO CO., LTD.
Chiyoda-ku, Tokyo 100-8185 (JP)**

(72) Inventors:
• **Nakagiri, Ryusuke,
Tsukuba Research Laboratories
Tsukuba-shi, Ibaraki 305-0841 (JP)**

• **Kamiya, Toshikazu,
Tsukuba Research Laboratories
Tsukuba-shi, Ibaraki 305-0841 (JP)**
• **Hashizume, Erika,
Tsukuba Research Laboratories
Tsukuba-shi, Ibaraki 305-0841 (JP)**
• **Sakai, Yasushi, Foods & Liquors Research Lab.
Inashiki-gun, Ibaraki 300-0398 (JP)**
• **Kayahashi, Shun,
Tsukuba Research Laboratories
Tsukuba-shi, Ibaraki 305-0841 (JP)**

(74) Representative: **VOSSIUS & PARTNER
Siebertstrasse 4
81675 München (DE)**

(54) **Liver function protecting or improving agent**

(57)     The present invention provides a liver function protecting or improving agent, foods and drinks or feeds having liver function protecting or improving activity, and additives for foods and drinks or feeds having liver function protecting or improving activity, which comprise a plant of the family Saxifragaceae or an extract of the plant. Also provided is a method of screening for liver function protecting or improving agents.

Printed by Jouve, 75001 PARIS (FR)

**Description**

The present invention relates to a liver function protecting or improving agent, foods and drinks or feeds having liver function protecting or improving activity, additives for foods and drinks or for feeds having liver function protecting or improving activity, a method of screening for liver function protecting or improving agents, and a method of protecting or improving the animal liver function.

The liver is an important organ which has various functions such as metabolic regulation and storage of sugar, protein and lipid which are three major nutrients, and decomposition and detoxification of substances unnecessary to the body. These functions suffer acute or chronic disorders due to an excessive intake of alcohol, viral infection, bad eating habits, stress, smoking, etc. The advance of these disorders brings about diseases such as acute hepatitis, chronic hepatitis, hepatic cirrhosis, alcoholic fatty liver, hepatitis B and liver cancer.

When liver cells are damaged by virus, alcohol, etc., enzymes such as aspartate aminotransferase (glutamicoxaloacetic transaminase, hereinafter abbreviated as GOT) and alanine aminotransferase (glutamic-pyruvic transaminase, hereinafter abbreviated as GPT) in the cells leak into the blood, which raises the values indicating the activities of these enzymes. Accordingly, the levels of GOT and GPT activities in the blood are known to indicate the levels of the liver function disorders.

Known drugs used for the protection or treatment of the liver function disorders include antiviral agents such as acyclovir, immunosuppressive agents (Journal of Clinical and Experimental Medicine, Vol. 171, No. 14, 957-1158, Ishiyaku Pub., Inc., 1994), and glutathione (Protein, Nucleic Acid and Enzyme, Vol. 33, No. 9, 1625-1631, Kyoritsu Shuppan Co., Ltd., 1988). Foods and drinks which are recognized to be effective for protecting, strengthening and improving the liver function include turmeric, milk thistle, sesame lignan, oyster extract and liver extract (Food Style 21, Vol. 2, No. 12, Shokuhin Kagaku Shinbunsha, 1998).

Hydrangea macrophylla Seringe var. Thunbergii Makino, which is a plant of the family Saxifragaceae, is a species akin to hydrangea and said to have been developed in the process of breeding Hydrangea macrophylla Seringe var. acuminata. Hydrangeae Dulcis Folium, which is prepared by fermenting leaves and branch ends of Hydrangea macrophylla Seringe var. Thunbergii Makino, followed by drying, is known as a crude drug produced in Japan.

Hydrangeae Dulcis Folium and an extract of Hydrangeae Dulcis Folium have been conventionally used as starting materials for correctives (sweeteners) in pills and for mouth refreshers (The Japanese Pharmacopoeia, 12th revision, D-31-33, Hirokawa Pub., Co., Ltd., 1991). The extract of Hydrangeae Dulcis Folium, which is a food additive (sweetener), is obtained by extracting leaves of Hydrangeae Dulcis Folium with water. It is used in the form of a liquid or a powder obtained by concentration, and its sweetening component is phyllodulcin (Existing Additives and Natural Flavors, Food Materials "Natural Products Handbook" 14th edition, Shokuhin to Kagaku, 1998).

Hydrangeae Dulcis Folium is known to have anti-Coccidium activity, anti-fungal activity, anti-ulcer activity, anti-allergic activity, hypercholesterolemia suppressing activity, anti-periodontal bacteria activity, anti-oxidation activity, and the like (Summary of Lectures at the 2nd Symposium on Medicines and Foods, p. 85, 1999). The extract of Hydrangeae Dulcis Folium is known to have cholagoic activity (Journal of the Pharmaceutical Society of Japan, Vol. 114, No. 6, 401-413, 1994). The extract of Hydrangeae Dulcis Folium is also known to exhibit suppressing activity on in vitro lipid peroxidation reaction of liver microsomes [Natural Medicines, 49(1), 84-87, 1995].

However, there has been no report that plants of the family Saxifragaceae such as Hydrangeae Dulcis Folium and Saxifraga stolonifera Meerb. have liver function protecting or improving activity.

Screening methods for liver function protecting or improving agents are known which use rodents having the blood GPT and GOT levels raised by administration of D-galactosamine [Journal of Nutrition, 129, 1361 (1999)], acetaminophen [Planta Medica, 55, 417 (1989)] and carbon tetrachloride [Fundamental & Clinical Pharmacology, 3, 183 (1989)].

On the other hand, it is known that GPT and GOT activities in the blood can be enhanced by administration of ethanol, followed by administration of lipopolysaccharide [Gastroenterology, 115, 443 (1998)]. By this method, hepatopathy can be induced in a shorter time than by the method in which alcohol alone is administered. However, no report has been made on the application of this method to the screening for liver function protecting or improving agents.

A strong need exists for the development of pharmaceutical agents which effectively prevent or treat liver diseases and of health foods and drinks or animal feeds which can prevent or treat hepatopathy by daily intake.

The present invention provides a liver function protecting or improving agent, foods and drinks or feeds having liver function protecting or improving activity, additives for foods and drinks or for feeds having liver function protecting or improving activity, a method of screening for liver function protecting or improving agents, and a method of protecting or improving the animal liver function.

The present invention relates to the following (1) to (51).

(1) A liver function protecting or improving agent which comprises a plant of the family Saxifragaceae or an extract of the plant as an active ingredient.

(2) The liver function protecting or improving agent according to (1), wherein the plant of the family <u>Saxifragaceae</u> belongs to the genus <u>Saxifraga.</u>

(3) The liver function protecting or improving agent according to (2), wherein the plant belonging to the genus <u>Saxifraga</u> is <u>Saxifraga stolonifera</u> Meerb.

(4) The liver function protecting or improving agent according to (1), wherein the plant of the family <u>Saxifragaceae</u> belongs to the genus <u>Hydrangea.</u>

(5) The liver function protecting or improving agent according to (4), wherein the plant belonging to the genus <u>Hydrangea</u> is <u>Hydrangea macrophylla</u> Seringe var. Thunbergii Makino or Hydrangeae Dulcis Folium.

(6) The liver function protecting or improving agent according to any of (1) to (5), wherein the extract of the plant of the family <u>Saxifragaceae</u> is an alcoholic extract of the residue of an aqueous medium extract of the plant of the family <u>Saxifragaceae.</u>

(7) The liver function protecting or improving agent according to any of (1) to (6), which is administered orally.

(8) The liver function protecting or improving agent according to any of (1) to (7), wherein the liver function is a function affected by alcohol.

(9) A food and/or drink which comprises a plant of the family <u>Saxifragaceae</u> or an extract of the plant.

(10) The food and/or drink according to (9), which is useful for the protection or improvement of liver function.

(11) The food and/or drink according to (10), wherein the liver function is a function affected by alcohol.

(12) The food and/or drink according to any of (9) to (11), wherein the plant of the family <u>Saxifragaceae</u> belongs to the genus <u>Saxifraga.</u>

(13) The food and/or drink according to (12), wherein the plant belonging to the genus <u>Saxifraga</u> is <u>Saxifraga stolonifera</u> Meerb.

(14) The food and/or drink according to any of (9) to (11), wherein the plant of the family <u>Saxifragaceae</u> belongs to the genus <u>Hydrangea.</u>

(15) The food and/or drink according to (14), wherein the plant belonging to the genus <u>Hydrangea</u> is <u>Hydrangea macrophylla</u> Seringe var. Thunbergii Makino or Hydrangeae Dulcis Folium.

(16) The food and/or drink according to any of (9) to (15), wherein the extract of the plant of the family <u>Saxifragaceae</u> is an alcoholic extract of the residue of an aqueous medium extract of the plant of the family <u>Saxifragaceae.</u>

(17) A feed which comprises a plant of the family <u>Saxifragaceae</u> or an extract of the plant.

(18) The feed according to (17), which is useful for the protection or improvement of liver function.

(19) The feed according to (18), wherein the liver function is a function affected by alcohol.

(20) The feed according to any of (17) to (19), wherein the plant of the family <u>Saxifragaceae</u> belongs to the genus <u>Saxifraga.</u>

(21) The feed according to (20), wherein the plant belonging to the genus <u>Saxifraga</u> is <u>Saxifraga stolonifera</u> Meerb.

(22) The feed according to any of (17) to (19), wherein the plant of the family <u>Saxifragaceae</u> belongs to the genus <u>Hydrangea.</u>

(23) The feed according to (22), wherein the plant belonging to the genus <u>Hydrangea</u> is <u>Hydrangea macrophylla</u> Seringe var. Thunbergii Makino or Hydrangeae Dulcis Folium.

(24) The feed according to any of (17) to (23), wherein the extract of the plant of the family Saxifragaceae is an alcoholic extract of the residue of an aqueous medium extract of the plant of the family Saxifragaceae.

(25) An additive for foods and drinks having liver function protecting or improving activity, which comprises a plant of the family Saxifragaceae or an extract of the plant.

(26) The additive for foods and drinks according to (25), wherein the liver function is a function affected by alcohol.

(27) The additive for foods and drinks according to (25) or (26), wherein the plant of the family Saxifragaceae belongs to the genus Saxifraga.

(28) The additive for foods and drinks according to (27), wherein the plant belonging to the genus Saxifraga is Saxifraga stolonifera Meerb.

(29) The additive for foods and drinks according to (25) or (26), wherein the plant of the family Saxifragaceae belongs to the genus Hydrangea.

(30) The additive for foods and drinks according to (29), wherein the plant belonging to the genus Hydrangea is Hydrangea macrophylla Seringe var. Thunbergii Makino or Hydrangeae Dulcis Folium.

(31) The additive for foods and drinks according to any of (25) to (30), wherein the extract of the plant of the family Saxifragaceae is an alcoholic extract of the residue of an aqueous medium extract of the plant of the family Saxifragaceae.

(32) A feed additive having liver function protecting or improving activity, which comprises a plant of the family Saxifragaceae or an extract of the plant.

(33) The feed additive according to (32), wherein the liver function is a function affected by alcohol.

(34) The feed additive according to (32) or (33), wherein the plant of the family Saxifragaceae belongs to the genus Saxifraga.

(35) The feed additive according to (34), wherein the plant belonging to the genus Saxifraga is Saxifraga stolonifera Meerb.

(36) The feed additive according to (32) or (33), wherein the plant of the family Saxifragaceae belongs to the genus Hydrangea.

(37) The feed additive according to (36), wherein the plant belonging to the genus Hydrangea is Hydrangea macrophylla Seringe var. Thunbergii Makino or Hydrangeae Dulcis Folium.

(38) The feed additive according to any of (32) to (37), wherein the extract of the plant of the family Saxifragaceae is an alcoholic extract of the residue of an aqueous medium extract of the plant of the family Saxifragaceae.

(39) A method of screening for liver function protecting or improving agents, which comprises administering an alcohol and then a lipopolysaccharide to an animal to raise the blood GPT or GOT level of the animal, administering a test substance to the animal, and estimating the activity of the test substance to lower the blood GPT or GOT level of the animal.

(40) The method according to (39), wherein the animal is a mammal.

(41) The method according to (39) or (40), wherein the lipopolysaccharide is derived from a microorganism belonging to the group of enteric bacteria.

(42) The method according to any of (39) to (41), wherein the liver function is a function affected by alcohol.

(43) A method of protecting or improving liver function in an animal, which comprises feeding the animal with the liver function protecting or improving agent according to any of (1) to (8) or the feed according to any of (17) to (24).

(44) The method according to (43), wherein the animal is selected from the group consisting of livestock, poultry and cultivated fish.

(45) A food, drink or feed for the protection or improvement of liver function which comprises a plant of the family Saxifragaceae or an extract of the plant as an active ingredient.

(46) The food, drink or feed according to (45), wherein the plant of the family Saxifragaceae belongs to the genus Saxifraga.

(47) The food, drink or feed according to (46), wherein the plant belonging to the genus Saxifraga is Saxifraga stolonifera Meerb.

(48) The food, drink or feed according to (45), wherein the plant of the family Saxifragaceae belongs to the genus Hydrangea.

(49) The food, drink or feed according to (48), wherein the plant belonging to the genus Hydrangea is Hydrangea macrophylla Seringe var. Thunbergii Makino or Hydrangeae Dulcis Folium.

(50) The food, drink or feed according to any of (45) to (49), wherein the extract of the plant of the family Saxifragaceae is an alcoholic extract of the residue of an aqueous medium extract of the plant of the family Saxifragaceae.

(51) The food, drink or feed according to any of (45) to (50), wherein the liver function is a function affected by alcohol.

In the present invention, the term "protection of liver function" is intended to include the protection of liver function from various disorders and the prevention of liver function disorders. The term "improvement of liver function" is intended to include the improvement or cure of disordered liver function and the improvement or enhancement of liver function.

In the present invention, the term "plants of the family Saxifragaceae" means plants classified under the scientific name Saxifragaceae [Makino's Illustrated Flora in Color, 40th edition, Hokuryukan (June 10, 1984), Makino's New Illustrated Flora of Japan, 1st edition, Hokuryukan (May 10, 1983)].

The plants of the family Saxifragaceae include plants of the genera Astilbe, Rodgersia, Saxifraga, Tanakaea, Aceriphyllum, Boykinia, Chrysosplenium, Tiarell, Mitella, Parnassia, Philadelphus, Deutzia, Platycrater, Hydrangea, Schizophragma, Cardiandra, Deinanthe, Itea, Ribes and Kirengeshoma, and plants bred from these plants. Preferred are plants belonging to the genera Hydrangea and Saxifraga and those bred therefrom which have enhanced liver function protecting or improving activity.

Examples of the plants belonging to the genus Astilbe are Astilbe microphylla Knoll, Astilbe thunbergii Miq., Astilbe odontophylla Miq., Astilbe japonica Miq. and Astilbe simplicifolia Makino.

An example of the plants belonging to the genus Rodgersia is Rodgersia podophylla A. Gray.

Examples of the plants belonging to the genus Saxifraga are Saxifraga stolonifera Meerb., Saxifraga nipponica Makino, Saxifraga fortunei Hook., Saxifraga cortusaefolia Sieb. et Zucc., Saxifraga japonica Boiss., Saxifraga fusca Maxim., Saxifraga sendaica Maxim. var. laciniata Nakai, Saxifraga merkii Fisch. var. idsuroei Engl., Saxifraga laciniata Nakai et Takeda, Saxifraga bronchialalis L., Saxifraga cernua L. and Saxifraga sachalinensis Fr. Schm. Preferred is Saxifraga stolonifera Meerb.

An example of the plants belonging to the genus Tanakaea is Tanakaea radicans Franch. et Sav.

An example of the plants belonging to the genus Aceriphyllum is Aceriphyllum rossii Engler.

Examples of the plants belonging to the genus Boykinia are Boykinia lycoctonifolia Engl. and Boykinia tellimoides Engl. et Irmsch. Examples of the plants belonging to the genus Chrysosplenium are Chrysosplenium qrayanum Maxim., Chrysosplenium stamineum Franch, Chrysosplenium japonicum Makino, Chrysosplenium flagelliferum Fr. Schm., Chrysosplenium macrostemon Maxim. and Chrysosplenium sphaerospermum Maxim.

An example of the plants belonging to the genus Tiarella is Tiarella polyphylla Don.

Examples of the plants belonging to the genus Mitella are Mitella japonica Miq. and Mitella pauciflora Rosend.

Examples of the plants belonging to the genus Parnassia are Parnassia palustris L., Parnassia alpicola Makino, Parnassia foliosa Hook. f. et Thoms. var. nummularia Nakai.

An example of the plants belonging to the genus Philadelphus is Philadelphus satsumi Sieb.

Examples of the plants belonging to the genus Deutzia are Deutzia crenata Sieb. et Zucc., Deutzia sieboldiana Maxim., Deutzia gracilis Sieb. et. Zucc., Deutzia maximowicziana Makino, Deutzia uniflora Shirai and Deutzia gracilis Sieb. et Zucc. var. nagurai Makino.

An example of the plants belonging to the genus Platycrater is Platycrater serrata Makino.

Examples of the plants belonging to the genus Hydrangea are Hydrangea macrophylla Seringe, Hydrangea macrophylla Seringe var. otaksa Makino, Hydrangea macrophylla Seringe subsp. serrata Makino var. japonica Makino, Hydrangea macrophylla Seringe var. acuminata, Hydrangea macrophylla Seringe var. Thunbergii Makino, Hydrangeae Dulcis Folium, Hydrangea scandens Seringe, Hydrangea hirta Sieb. et Zucc., Hydrangea involucrata Sieb., Hydrangea sikokiana Maxim., Hydrangea paniculata Sieb., Hydrangea petiolaris Sieb. et Zucc., Hydrangea macrophylla Seringe subsp. serrata Makino var. amoena Makino, and Hydrangea macrophylla Seringe subsp. serrata Makino var. angustata Makino. Preferred are Hydrangea macrophylla Seringe var. Thunbergii Makino and Hydrangeae Dulcis Folium, and specifically preferred is Hydrangeae Dulcis Folium.

An example of the plants belonging to the genus Schizophragma is Schizophragma hydrangeoides Sieb. et Zucc.

An example of the plants belonging to the genus Cardiandra is Cardiandra alternifolia Sieb. et Zucc.

An example of the plants belonging to the genus Deinanthe is Deinanthe bifida Maxim.

An example of the plants belonging to the genus Itea is Itea japonica Oliver.

Examples of the plants belonging to the genus Ribes are Ribes sinanense F. Maekawa, Ribes grossularia L., Ribes latifolium Jancz., Ribes rubrum L., Ribes japonicum Maxim., Ribes sachalinense Nakai, Ribes fasciculatum Sieb. et Zucc., Ribes alpinum L. var. japonicum Maxim., and Ribes ambiguum Maxim.

An example of the plants belonging to the genus Kirengeshoma is Kirengeshoma palmata Yatabe.

The term "plants" of the present invention also includes leaves, flowers, branches, stalks, fruits, roots, seeds, cultured cells or organs, callus, etc. of wild plants, cultivated plants or plants grown by culturing such as tissue culture, which are used as such or after being treated physically, chemically or biologically.

The physical or chemical treatment includes drying such as sun-drying, air-drying and freeze-drying, and disruption with a blender, a homogenizer, a ball mill, etc. The physically or chemically treated matters include dried matters, freeze-dried matters and disrupted matters. The biological treatment includes fermentation and the biologically treated matters include fermented matters.

The extracts of the plants include extracts obtained from the above-described plants by various methods of extraction. Examples of the methods of extraction are extraction with various solvents and supercritical fluid extraction. Extracts may further be treated by various methods of solid-liquid separation such as sedimentation, cake filtration, clear filtration, centrifugal filtration, centrifugal sedimentation, separation by compression and filter press, various concentration methods, various drying methods, methods of making various preparations such as granulation and pulverization, and various purification methods.

The purification methods include fractionation with a solvent, column chromatography and recrystallization. Specifically preferred is column chromatography using various carriers such as DIAION HP-20 (Mitsubishi Chemical Corporation) and Sephadex LH-20 (Pharmacia).

Examples of the concentration and drying methods are freeze-drying, natural drying, hot air-drying, air-drying, spray drying, drying under reduced pressure, sun-drying, vacuum drying, fluidized-bed drying, foam-bed drying, drum drying, ultrasonic drying and electromagnetic wave drying. Preferred are spray drying and freeze-drying.

In the step of extraction and treatment of an extract, an antioxidant, a preservative, etc. may be added.

As the solvent for extraction, any solvent which can extract a substance exhibiting liver function protecting or improving activity of the present invention can be used. Suitable solvents include aqueous media such as water, distilled water, deionized water, an aqueous solution of inorganic salt and buffer, monovalent alcohols such as methanol, ethanol, propanol and butanol, polyvalent alcohols such as propylene glycol and glycerol, and organic solvents such as hexane, toluene, petroleum ether, benzene, ethyl acetate, chloroform, dichloromethane, 1,1,2-trichloroethene, dimethyl sulfoxide and acetone. Preferred are aqueous media and alcohols.

Examples of the buffers are phosphate buffer and citrate buffer. Examples of the aqueous solutions of inorganic salts are those of sodium chloride, potassium chloride and calcium chloride.

Preferred alcohols are monovalent alcohols and a preferred monovalent alcohol is ethanol.

These solvents can be used alone or as a mixture. As the mixed solvent, water-containing alcohols are preferred. Water-containing monovalent alcohols are more preferred and water-containing ethanol is specifically preferred. The water content is preferably 70% or lower, more preferably 40% or lower.

As the solvent, supercritical fluid carbon dioxide may also be employed.

For extraction, the solvent is used in an amount of 0.1 to 10000 parts by weight, preferably 1 to 100 parts by weight for 1 part by weight of a plant. There is no specific restriction as to the temperature for extraction, but it is preferably 0 to 100°C, more preferably 20 to 90°C. There is no specific restriction as to the time for extraction, but it is preferably one minute to one week, more preferably 30 minutes to one day.

Specifically, extraction from the plants of the family Saxifragaceae is preferably carried out by extracting the above-described plants of the family Saxifragaceae, as such or after the physical, chemical or biological treatment, with an aqueous medium and then extracting the residues of the aqueous medium extracts of the plants with alcohol or water-containing alcohol.

There is no specific restriction as to the aqueous medium, but water, pure water and deionized water are preferred. The temperature for extraction with an aqueous medium, alcohol or water-containing alcohol is not specifically restricted, but is preferably 0 to 100°C, more preferably 20 to 90°C. The time for extraction is not specifically restricted, but is preferably one minute to one week, more preferably 30 minutes to one day. It is preferred to use the plants of the family Saxifragaceae after drying or fermenting treatment.

There is no specific restriction as to the apparatus to be employed for extraction. Preferred apparatuses include an apparatus designed for effective extraction, a stirrer, a reflux condenser, a Soxhlet extractor, a homogenizer, a shaker and a ultrasonic generator.

The liver function protecting or improving agent of the present invention comprises a plant of the family Saxifragaceae or an extract thereof prepared by the above-described method, and if necessary, may comprise one or more pharmaceutically acceptable carriers, and further, an active ingredient for another treatment.

The present pharmaceutical composition is prepared by mixing a plant of the family Saxifragaceae or an extract thereof with a carrier, as may be required, according to any of the methods well known in the field of pharmaceutics.

It is desirable to administer the composition by the route which is most effective for the treatment. Suitable administration routes include oral administration and non-oral administration such as intravenous administration, intraperitoneal administration or subcutaneous administration. Preferred is oral administration.

The composition is administered in the form of tablets, powders, granules, pills, suspensions, emulsions, infusa, capsules, syrups, injections, liquids, elixirs, extracts, tinctures, fluid extracts, etc.

Extracts, tinctures, fluid extracts, etc. suitable for oral administration can be prepared by extracting a plant of the family Saxifragaceae with water, ethanol or a mixture of water and ethanol, if necessary followed by concentration.

Liquid preparations suitable for oral administration such as syrups can be prepared using carriers such as water, sugars (e.g. sucrose, sorbitol and fructose), glycols (e.g. polyethylene glycol and propylene glycol), oils (e.g. sesame oil, olive oil and soybean oil), antiseptics (e.g. p-hydroxybenzoate), paraoxybenzoic acid derivatives (e.g. methyl paraoxybenzoate), preservatives (e.g. sodium benzoate) and flavors (e.g. strawberry flavor and peppermint).

Tablets, powders, granules, etc. suitable for oral administration can be prepared using sugars such as lactose, white sugar, glucose, sucrose, mannitol and sorbitol, starch such as potato starch, wheat starch and corn starch, inorganic substances such as calcium carbonate, calcium sulfate, sodium hydrogencarbonate and sodium chloride, excipients such as crystalline cellulose and plant powders (e.g. licorice powder and gentian powder), disintegrating agents such as starch, agar, gelatin powder, crystalline cellulose, carmellose sodium, carmellose calcium, calcium carbonate, sodium hydrogencarbonate and sodium alginate, lubricants such as magnesium stearate, talc, hydrogenated vegetable oil, macrogol and silicone oil, binders such as polyvinyl alcohol, hydroxypropyl cellulose, methyl cellulose, ethyl cellulose, carmellose, gelatin and starch paste, surfactants such as fatty acid ester, plasticizers such as glycerin, and the like.

Preparations appropriate for non-oral administration such as injections comprise, preferably, a sterilized aqueous agent containing an active compound which is isotonic to the recipient's blood. In the case of an injection, for example, an injectable solution is prepared using a carrier such as a salt solution, a glucose solution, or a mixture of a salt solution and a glucose solution.

The above-described antiseptics, preservatives, surfactants, etc. can also be employed in non-oral preparations.

The dose of the liver function protecting or improving agent of the present invention will vary depending on the administration route, the age and body weight of a patient and the symptom and degree of the disease to be treated, without specific restriction. For instance, when the agent is orally administered to an adult, it is suitable to administer the agent in an amount of 0.01 mg to 50 g, preferably 0.05 mg to 10 g in terms of dry weight of a plant of the family Saxifragaceae or an extract of the plant once to several times per day. In the case of non-oral administration such as intravenous administration, it is suitable to administer the agent in an amount of 0.001 mg to 50 g, preferably 0.01 mg to 10 g in terms of dry weight of a plant of the family Saxifragaceae or an extract of the plant once to several times per day. In the case of administration to an animal, the dose will vary depending on the age and kind of the animal and the symptom and degree of the disease, without specific restriction. It is generally suitable to administer the agent in an amount of 0.1 μg to 10 g, preferably 1 μg to 1 g per kg once to several times per day. In the case of non-oral administration such as intravenous administration, it is suitable to administer the agent in an amount of 0.01 μg to 10 g, preferably 1 μg to 1 g per kg once to several times per day. However, the dose may vary depending upon the above-mentioned conditions.

The foods, drinks or feeds having liver function protecting or improving activity which comprise a plant of the family Saxifragaceae or an extract of the plant include those prepared by adding to foods drinks or feeds a plant of the family Saxifragaceae or an extract of the plant of the present invention in a process for producing ordinary foods, drinks or feeds. The foods, drinks or feeds of the present invention may be processed by molding and granulating methods.

The molding and granulating methods include granulating methods such as fluidized bed granulation, stirring granulation, extrusion granulation, rolling granulation, air stream granulation, compression molding granulation, disruption granulation, spray granulation and blasting granulation, coating methods such as pan coating, fluidized bed coating and dry coating, plumping methods such as puff drying, excess steam method, foam mat method and microwave heating method, and extrusion methods using an extruding granulator and an extruder.

The foods, drinks or feeds for the protection or improvement of liver function comprising a plant of the family Saxifragaceae or an extract of the plant as an active ingredient include plants of the family Saxifragaceae and extracts of the plants themselves which can be used as foods, drinks or feeds; and foods, drinks or feeds obtained by adding a plant of the family Saxifragaceae or an extract of the plant to foods, drinks, feeds or their starting materials.

There is no specific restriction as to the foods, drinks, feeds, or their starting materials to which a plant of the family Saxifragaceae or an extract of the plant is added. Useful foods, drinks, feeds, or their starting materials include both those which comprise a plant of the family Saxifragaceae or an extract of the plant and those which do not substantially comprise a plant of the family Saxifragaceae or an extract of the plant.

The liver function protecting or improving activity of the foods and drinks or feeds which comprise a plant of the family Saxifragaceae or an extract of the plant can be enhanced by adding a plant of the family Saxifragaceae or an extract of the plant thereto.

There is no specific restriction as to the amount of a plant of the family Saxifragaceae or an extract of the plant of the present invention to be added to foods, drinks or feeds, so long as it gives a content which enables foods and drinks or feeds to exhibit liver function protecting or improving activity. For instance, it is suitable to add a plant of the family Saxifragaceae or an extract of the plant in an amount of 0.001 to 100%, preferably 0.01 to 100%, more preferably 0.1-100%, in terms of dry weight.

Examples of the foods and drinks comprising a plant of the family Saxifragaceae or an extract of the plant are juice, soft drinks, soup, tea, dairy products (e.g. lactic acid bacteria beverages, fermented milk, ice cream, butter, cheese, yogurt, processed milk and skim milk), meat products (e.g. ham, sausage and hamburger), fish products, egg products (e.g. fried or steamed foods made of beaten eggs), confectionery (e.g. cookies, jelly, snacks and chewing gum), bread, noodles, pickles, smoked fish and meat, dry fish, preserved foods boiled down with soy and seasonings which comprise a plant of the family Saxifragaceae or an extract of the plant.

The foods and drinks may be in any of the forms such as a powder food, a sheet-shaped food, a bottled food, a canned food, a retort pouched food, a capsule food, a tablet food, a liquid food and a liquid nutrient food.

The foods and drinks of the present invention are used as health foods and drinks and functional foods and drinks for the protection or improvement of liver function.

There is no specific restriction as to the intake of foods and drinks of the present invention having liver function protecting or improving activity which comprise a plant of the family Saxifragaceae or an extract of the plant as an active ingredient. It is generally suitable to take a plant of the family Saxifragaceae or an extract of the plant in an amount of 0.1 to 50 g, preferably 0.5 to 10 g (dry weight) per adult per day for one day to one year, preferably 2 weeks to 3 months. This intake is merely a typical example and can be appropriately adjusted according to the recipient's condition, age, weight, etc.

The feeds of the present invention can be obtained, for example, by adding a plant of the family Saxifragaceae or an extract of the plant to feed materials.

The feeds of the present invention include any feeds having liver function protecting or improving activity on animals such as mammals, birds, reptiles, amphibians and fish. Examples of the feeds are feed for pets such as dogs, cats and mice, feed for livestock such as cows and pigs, feed for poultry such as hens and turkeys, and feed for cultivated fish such as sea breams and young yellowtails.

The feeds of the present invention can be prepared by appropriately mixing a plant of the family Saxifragaceae or an extract thereof with feed materials. The feed materials include grains, bran, vegetable oil cakes, animal feed materials, other feed materials and purified products.

Examples of the grains are milo, wheat, barley, oats, rye, brown rice, buckwheat, foxtail millet, broomcorn millet, Japanese millet, corn and soybean.

Examples of the bran are rice bran, defatted rice bran, wheat bran, wheat middlings, wheat germ, corn bran and corn germ.

Examples of the vegetable oil cakes are soybean oil cake, soybean flower, linseed oil cake, cottonseed oil cake, peanut oil cake, safflower oil cake, coconut oil cake, palm oil cake, sesame oil cake, sunflower oil cake, rapeseed oil cake, kapok oil cake and mustard seed oil cake.

Examples of the animal feed materials are fish meal (e.g. northern ocean meal, imported meal, whole meal and coastal meal), fish soluble, meat meal, meat and bone meal, blood powder, degradated hair, bone meal, treated by-products for livestock, feather meal, silkworm pupa, skim milk, casein and dry whey.

Examples of other feed materials are stalks and leaves of plants (e.g. alfalfa, hay cube, alfalfa leaf meal and powder of false acacia), processed industrial by-products of corn (e.g. corn gluten, meal, corn gluten feed and corn steep liquor), processed starch products (e.g. starch), sugar, fermentation industrial products (e.g. yeast, beer cake, malt root, alcohol cake and soy sauce cake), agricultural by-products (e.g. processed citrus fruit cake, tofu cake, coffee cake and cocoa cake) and others (e.g. cassava, broad bean, guar meal, seaweeds, krill, spirulina, chlorella and minerals).

Examples of the purified products are proteins (e.g. casein and albumin), amino acids, sugars (e.g. starch, cellulose, sucrose and glucose), minerals and vitamins.

There is no specific restriction as to the intake of the feeds of the present invention having liver function protecting or improving activity which comprise a plant of the family Saxifragaceae or an extract of the plant as an active ingredient. It is generally suitable to take a plant of the family Saxifragaceae or an extract of the plant in an amount of 0.1 mg to 50 g, preferably 0.5 mg to 10 g (dry weight) per kg per day for one day to one year, preferably 2 weeks to 3 months. This intake is merely a typical example and can be appropriately adjusted according to the kind, age, weight, etc. of an animal to be fed.

The additives for foods and drinks or feeds having liver function protecting or improving activity which comprise a plant of the family Saxifragaceae or an extract of the plant as an active ingredient comprise, as an active ingredient, a plant of the family Saxifragaceae or an extract thereof prepared according to the above-described method and may comprise, if necessary, ordinary additives employed in foods and drinks or feeds, for example, additives listed in Food Additives Indication Pocket Book (Japan Food Additives Association, Jan. 6, 1997) such as sweeteners, coloring agents, preservatives, thickening stabilizers, antioxidants, color developing agents, bleaching agents, fungicides, gum bases, bitter agents, enzymes, wax, sour agents, seasonings, emulsifiers, nutrient supplements, additional materials for preparation, flavors and spice extracts. The carriers mentioned in the above description of pharmaceutical compositions may also be added.

Examples of the sweeteners are aspartame, licorice, stevia, xylose and Momordica grosvenori. Examples of the coloring agents are carotenoid pigment, turmeric pigment, flavonoid, caramel pigment, oriental gromurell pigment, spirulina pigment, chlorophyll, red sweet potato pigment, red Chinese yam pigment, perilla pigment and blueberry pigment.

Examples of the preservatives are sodium sulfite, benzoic acid and benzoates, extract of Aralia cordata, Japanese Styrax benzoin extract, Rumpet roman extract, sorbic acid and sorbates, and propionic acid and propionates. Examples of the thickening stabilizers are gums such as gum arabic and xanthane gum, alginic acid and alginates, chitin, chitosan, aloe extract, guar gum, hydroxypropyl cellulose, casein sodium, corn starch, carboxymethyl cellulose, gelatin, agar, dextrin, methyl cellulose, polyvinyl alcohol, microfibrous cellulose, microcrystalline cellulose, seaweed cellulose, sodium polyacrylate, sodium polyphosphate, carrageenan, yeast cell wall, extract of konjac, nata de coco and mannan.

Examples of the antioxidants are vitamin C, sodium ethylenediaminetetraacetate, calcium ethylenediaminetetraacetate, erythorbic acid, oryzanol, catechin, quercetin, clove extract, enzyme-treated rutin, apple extract, sesame oil extract, dibutylhydroxytoluene, fennel extract, horseradish extract, water dropwort extract, tea extract, Tempeh extract, extract of Houttuynia cordata, tocotrienol, tocopherols, rapeseed oil extract, green coffee extract, sunflower seed, ferulic acid, butylhydroxyanisole, blueberry leaf extract, propolis extract, hego-ginkgo leave extract, hesperetin, pepper extract, garden balsam extract, gallic acid, myrica extract, eucalyptus extract and rosemary extract.

An example of the color developing agent is sodium nitrite and an example of the bleaching agent is sodium sulfite.

An example of the fungicide is orthophenylphenol.

Examples of the gum bases are methyl acetylricinoleate, Japanese lacquer wax, ester gum, elemi resin, urucury wax, ozokerite, opopanax resin, kauri gum, carnauba wax, guaiacum resin, gutta katiau, gutta hangkang, guttapercha, glycerin fatty acid ester, spermaceti, copaiba balsam, copal resin, gum, rice bran wax, sugar cane wax, shellac, jelutong, sucrose fatty acid ester, sorba, sorbitan fatty acid ester, talc, calcium carbonate, dammar resin, chicle, chilte, tunu, low-molecular gum, paraffin wax, fir balsam, propylene glycol fatty acid ester, powdered pulp, powdered rice husks, jojoba wax, polyisobutylene, polybutene, microcrystalline wax, mastic, massaranduba chocolate, beeswax, and calcium phosphate.

Examples of the bitter agents are isoalpha bitter acid, caffeine, kawaratake extract, cinchona extract, Amur cork extract, gentian extract, spice extracts, enzyme-treated naringin, Jamaica quassia extract, theobromine, naringin, bitter ash extract, warmwood extract, isodonis extract, himematsutake extract, borapet, methyl thioadenosine, litchi extract, olive tea, sour orange extract, hop extract and mugwort extract.

Examples of the enzymes or enzyme sources are amylase, trypsin, rennet and lactic acid bacteria.

Examples of the wax are Japanese lacquer wax and vegetable wax. Examples of the sour agents are adipic acid, itaconic acid, citric acid and citrates, succinic acid and succinates, sodium acetate, tartaric acid and tartrates, carbon dioxide, lactic acid, phytic acid, fumaric acid, malic acid and phosphoric acid. Examples of the seasonings are amino acids such as asparagine, aspartic acid, glutamic acid, glutamine, alanine, isoleucine, glycine, serine, cystine, tyrosine, leucine and proline, nucleic acids such as sodium inosinate, sodium uridylate, sodium guanylate, sodium cytidylate, calcium ribonucleotide and sodium ribonucleotide, organic acids such as citric acid and succinic acid, potassium chloride, sodium solution of low salt content prepared from salt lake water, crude potassium chloride from sea water, whey salt, tripotassium phosphate, dipotassium hydrogenphosphate, potassium dihydrogenphosphate, disodium hydrogenphosphate, sodium dihydrogenphosphate, trisodium phosphate and chlorella extract.

Examples of the nutrient supplements are zinc salts, vitamin C, various amino acids, 5-adenylic acid, iron chloride, hesperidin, various kinds of burnt calcium, various kinds of unburnt calcium, dibenzoylthiamine, calcium hydroxide, calcium carbonate, thiamine hydrochloride, dunaliella carotene, tocopherol, nicotinic acid, carrot carotene, palm oil carotene, calcium pantothenate, vitamin A, hydroxyproline, calcium dihydrogenpyrophosphate, iron (II) pyrophosphate, iron (III) pyrophosphate, ferritin, heme iron, menaquinone, folic acid and riboflavin. Examples of the additional materials for preparation are processing aids such as acetone and ion exchange resin, extract of fig leaf, extract of rice straw ash, kaolin, glycerin fatty acid ester, mulberry extract, bone ash, perilla extract, ginger extract, various tannins, Phaffia color, grape seed extract and ethanol.

These additives can also be added to the above-described liver function protecting or improving agent and foods and drinks or feeds having liver function protecting or improving activity.

The term "liver function" as used herein means every function of the liver and there is no limit as to the definition of the term. Specific examples of the liver functions are those relating to blood and circulation such as storage of blood (adjustment of the amount of circulating blood, etc.), treatment of blood pigments (discharge of hemoglobin, etc.), formation of bile, enterohepatic circulation of bile pigments, and synthesis of plasma proteins (e.g. acute phase proteins, albumin, blood coagulation factors, steroid-binding proteins and other hormone-binding proteins), metabolic functions such as metabolism of nutrients and vitamins (e.g. glucose and other sugars, amino acids, lipids or fatty acids, cholesterol, lipoproteins, lipid-soluble vitamins and water-soluble vitamins), detoxification or decomposition functions such as inactivation of various substances (e.g. toxins, steroids such as estrogen and androsterone, and other hormones) and immune functions ["Seirigaku Tenbo" (View of Physiology), 19th edition (Mar. 31, 2000), "Atarashii Rinsho Eiyogaku" (New Study of Clinical Nutrition), 3rd revision (May 20, 2000)]. These functions all suffer damage from an excessive intake of alcohol.

Described below is a method of screening for liver function protecting or improving agents, which comprises administering an alcohol and then a lipopolysaccharide to an animal to raise the blood GPT or GOT level of the animal, administering a test substance to the animal, and estimating the activity of the test substance to lower the blood GPT or GOT level of the animal.

There is no specific restriction as to the animal to be used for screening so far as it is an animal whose blood GPT or GOT level rises after administration of alcohol followed by administration of lipopolysaccharide. Suitable animals include mammals such as mice, rats, rabbits, dogs and cats.

Test substances for screening can be administered to animals by various administration routes such as feeding, oral administration, intraperitoneal administration, intravenous administration and intramuscular administration. Preferred are feeding and oral administration. Test substances can be administered to animals *ad lib.* or at regular intervals. For instance, a test substance is administered as a mixture with feed or as such once to three times a day for one hour to 360 days, preferably one day to 2 months, more preferably 3 to 15 days.

Alcohol can be administered to animals by oral administration, intravenous administration, or the like. Preferred is oral administration. Administration of alcohol may be carried out before, simultaneously with or after the administration of a test substance, but is preferably carried out after the administration of a test substance.

The amount of alcohol to be administered is 2 to 10 g/kg of an animal, preferably 3 to 5 g/kg. Administration is preferably carried out using a sound, etc. Alcohol is administered, for example, once to 360 times, preferably once to 10 times, for one hour to 360 days, preferably one day to 2 months, more preferably 3 days to 15 days. Then, a lipopolysaccharide is intravenously injected to the animal in an amount of 0.15 to 15 mg/kg, preferably 3 to 6 mg/kg, one to 24 hours, preferably 3 to 9 hours, specifically 6 hours after the administration of alcohol. The blood GPT or GOT level is determined 12 to 36 hours, preferably 20 to 28 hours later. This GPT or GOT level is compared with that obtained by administrating alcohol and then lipopolysaccharide to an animal without administration of a test substance. Screening for liver function protecting or improving agents can be carried out by selecting substances which lower the GPT or GOT level raised by the administration of alcohol and lipopolysaccharide.

Suitable lipopolysaccharides include those extracted from Gram-negative bacteria according to known methods. Examples of the Gram-negative bacteria are photosynthetic bacteria such as those belonging to the genus Rhodopseudomonas, bacteria belonging to the genus Pseudomonas, enteric bacteria such as those belonging to the genera Escherichia and Salmonella, lithotrophic bacteria such as those belonging to the genera Nitrobacter and Thiobacillus, and methane-forming bacteria such as those belonging to the genus Neisseria. Of these lipopolysaccharides, preferred are those derived from enteric bacteria, specifically those belonging to the genus Escherichia.

The screening method of the present invention enables advantageous screening for preventing or treating agents for alcoholic hepatopathy.

There is no specific restriction as to the method of determination of the GPT or GOT level so long as it can determine the GPT or GOT level. Determination of the GPT level can be carried out, for example, by determining pyruvic acid formed from 2-oxoglutamic acid and alanine. Determination of pyruvic acid can be carried out, for example, by measuring the decrease in the absorbance at 340 nm caused by the reduction of pyruvic acid in the presence of lactate dehydrogenase and NADH.

Determination of the GOT level can be carried out, for example, by determining oxaloacetic acid formed from aspartic acid and 2-oxoglutaric acid. Determination of oxaloacetic acid can be carried out, for example, by measuring the decrease in the absorbance at 340 nm caused by the reduction of oxaloacetic acid in the presence of malate dehydrogenase and NADH.

According to the present invention, liver function protecting or improving agents can be selected by selecting substances which significantly lower the blood GPT or GOT level in animals raised by administration of alcohol followed by administration of lipopolysaccharide.

Certain embodiments of the present invention are illustrated in the following examples. These examples are not to be construed as limiting the scope of the invention.

The following substances were used in the examples. Powder of Hydrangeae Dulcis Folium (Shihira Shoten), Powder of <u>Saxifraga stolonifera</u> Meerb. (Shihira Shoten), D-Galactosamine (Wako Pure Chemical Industries, Ltd.), Collagenase (Sigma Chemical Co., Ltd.), Acetaminophen (Wako Pure Chemical Industries, Ltd.), Penicillin (Gibco), Streptomycin (Gibco), DMSO (Wako Pure Chemical Industries, Ltd.), Insulin (Wako Pure Chemical Industries, Ltd.), Dexamethasone (Wako Pure Chemical Industries, Ltd.), TNF-$\alpha$ (Wako Pure Chemical Industries, Ltd.), Waymouth's MB752/1 (Gibco), FBS (Gibco), Pine-dex #3 (Matsutani Chemical Industry Co., Ltd.), Iron (III) pyrophosphate (Kokusan Chemical Works Co., Ltd.)

Example 1 Production of a Freeze-dried Powder of a Water Extract of Hydrangeae Dulcis Folium

Dry powder of Hydrangeae Dulcis Folium (1 kg, Shihira Shoten) was extracted twice with 10 1 of distilled water at room temperature with stirring for one hour. The obtained extract was concentrated and freeze-dried to obtain 200 g of a freeze-dried powder of a water extract of Hydrangeae Dulcis Folium.

Example 2 Production of a Freeze-dried Powder of an Extract of Hydrangeae Dulcis Folium Extracted with a 60% Aqueous Solution of Ethanol

Dry powder of Hydrangeae Dulcis Folium (1 kg) was extracted twice with 10 1 of a 60% aqueous solution of ethanol at room temperature with stirring for one hour. The obtained extract was concentrated and freeze-dried to obtain 200 g of a freeze-dried powder of an extract of Hydrangeae Dulcis Folium extracted with a 60% aqueous solution of ethanol.

Example 3 Production of a Freeze-dried Powder of an Acetone Extract of Hydrangeae Dulcis Folium

Dry powder of Hydrangeae Dulcis Folium (1 kg) was extracted twice with 10 l of acetone at room temperature with stirring for one hour. The obtained extract was concentrated and freeze-dried to obtain 100 g of a freeze-dried powder of an acetone extract of Hydrangeae Dulcis Folium.

Example 4 Production of a Freeze-dried Powder of a Hot Water Extract of Hydrangeae Dulcis Folium

Dry powder of Hydrangeae Dulcis Folium (1 kg) was extracted with 10 1 of boiling distilled water for 30 minutes. The obtained extract was concentrated and freeze-dried to obtain 130 g of a freeze-dried powder of a hot water extract of Hydrangeae Dulcis Folium.

Example 5 Production of a Freeze-dried Powder of an Ethanol Extract of the Residue of a Water Extract of Hydrangeae Dulcis Folium

Dry powder of Hydrangeae Dulcis Folium (1 kg) was extracted with 20 l of distilled water at 40°C with stirring until the absorbance of the 1/200 dilution at 313 nm became 0.15. The obtained extract was filtered and the filtrate was removed to obtain the extract residue. The residue was extracted with 20 1 of 60% ethanol at 40°C with stirring until the absorbance of the 1/200 dilution at 313 nm became 0.22. The obtained extract was concentrated and freeze-dried to obtain 70 g (yield based on dry leaves: 7%) of a freeze-dried powder of an ethanol extract of the residue of a water extract of Hydrangeae Dulcis Folium.

Example 6 Production of a Freeze-dried Powder of an Acetone Extract of the Residue of a Water Extract of Hydrangeae Dulcis Folium

Dry powder of Hydrangeae Dulcis Folium (1 kg) was extracted with 20 1 of distilled water at 40°C with stirring until the absorbance of the 1/200 dilution at 313 nm became 0.15. The obtained extract was filtered and the filtrate was removed to obtain the extract residue. The residue was extracted with 20 l of acetone at 40°C with stirring until the absorbance of the 1/200 dilution at 313 nm became 0.22. The obtained extract was concentrated and freeze-dried to obtain 60 g (yield based on dry leaves: 6%) of a freeze-dried powder of an acetone extract of the residue of a water extract of Hydrangeae Dulcis Folium.

Example 7 Production of a Feed Containing 3% Freeze-dried Powder of Example 1

A feed having the following composition was prepared by mixing the ingredients.

| Sucrose (Kishida Chemical Co., Ltd.) | 20.0 wt% |
|---|---|
| Corn oil (Nacalai Tesque, Inc.) | 5.0 wt% |
| Choline bitartrate (Tokyo Kasei Kogyo Co., Ltd.) | 0.4 wt% |
| Corn starch (Nippon Starch Chemical Co., Ltd.) | 37.1 wt% |
| AIN-76 vitamin (Oriental Yeast Co., Ltd.) | 1.0 wt% |
| AIN-76 mineral (Oriental Yeast Co., Ltd.) | 3.5 wt% |
| Cellulose (Oriental Yeast Co., Ltd.) | 5.0 wt% |
| Casein (Wako Pure Chemical Industries, Ltd.) | 25.0 wt% |
| Powder produced in Example 1 | 3.0 wt% |

Example 8 Production of a Feed Containing 3% Freeze-dried Powder of Example 2

A feed having the following composition was prepared by mixing the ingredients.

| Sucrose (Kishida Chemical Co., Ltd.) | 20.0 wt% |
|---|---|
| Corn oil (Nacalai Tesque, Inc.) | 5.0 wt% |
| Choline bitartrate (Tokyo Kasei Kogyo Co., Ltd.) | 0.4 wt% |
| Corn starch (Nippon Starch Chemical Co., Ltd.) | 37.1 wt% |
| AIN-76 vitamin (Oriental Yeast Co., Ltd.) | 1.0 wt% |
| AIN-76 mineral (Oriental Yeast Co., Ltd.) | 3.5 wt% |
| Cellulose (Oriental Yeast Co., Ltd.) | 5.0 wt% |
| Casein (Wako Pure Chemical Industries, Ltd.) | 25.0 wt% |
| Powder produced in Example 2 | 3.0 wt% |

Example 9 Production of a Feed Containing 3% Freeze-dried Powder of Example 4

A feed having the following composition was prepared by mixing the ingredients.

| Sucrose (Kishida Chemical Co., Ltd.) | 20.0 wt% |
|---|---|
| Corn oil (Nacalai Tesque, Inc.) | 5.0 wt% |
| Choline bitartrate (Tokyo Kasei Kogyo Co., Ltd.) | 0.4 wt% |
| Corn starch (Nippon Starch Chemical Co., Ltd.) | 37.1 wt% |
| AIN-76 vitamin (Oriental Yeast Co., Ltd.) | 1.0 wt% |
| AIN-76 mineral (Oriental Yeast Co., Ltd.) | 3.5 wt% |
| Cellulose (Oriental Yeast Co., Ltd.) | 5.0 wt% |
| Casein (Wako Pure Chemical Industries, Ltd.) | 25.0 wt% |
| Powder produced in Example 4 | 3.0 wt% |

Example 10 Production of a Feed Containing 1% Freeze-dried Powder of Example 5

A feed having the following composition was prepared by mixing the ingredients.

| | |
|---|---|
| Sucrose (Kishida Chemical Co., Ltd.) | 20.0 wt% |
| Corn oil (Nacalai Tesque, Inc.) | 5.0 wt% |
| Choline bitartrate (Tokyo Kasei Kogyo Co., Ltd.) | 0.4 wt% |
| Corn starch (Nippon Starch Chemical Co., Ltd.) | 39.1 wt% |
| AIN-76 vitamin (Oriental Yeast Co., Ltd.) | 1.0 wt% |
| AIN-76 mineral (Oriental Yeast Co., Ltd.) | 3.5 wt% |
| Cellulose (Oriental Yeast Co., Ltd.) | 5.0 wt% |
| Casein (Wako Pure Chemical Industries, Ltd.) | 25.0 wt% |
| Powder produced in Example 5 | 1.0 wt% |

Comparative Example 1

A feed having the following composition was prepared by mixing the ingredients.

| | |
|---|---|
| Sucrose (Kishida Chemical Co., Ltd.) | 20.0 wt% |
| Corn oil (Nacalai Tesque, Inc.) | 5.0 wt% |
| Choline bitartrate (Tokyo Kasei Kogyo Co., Ltd.) | 0.4 wt% |
| Corn starch (Nippon Starch Chemical Co., Ltd.) | 40.1 wt% |
| AIN-76 vitamin (Oriental Yeast Co., Ltd.) | 1.0 wt% |
| AIN-76 mineral (Oriental Yeast Co., Ltd.) | 3.5 wt% |
| Cellulose (Oriental Yeast Co., Ltd.) | 5.0 wt% |
| Casein (Wako Pure Chemical Industries, Ltd.) | 25.0 wt% |

Example 11

The freeze-dried powder produced in Example 1 (5 g) was suspended in 500 ml of water. The resulting suspension was passed through a column packed with a hydrophobic adsorbent resin (Mitsubishi Chemical Corporation, DIAION HP-20, void volume: 100 ml), followed by elution with 250 ml of 33% methanol. Elution was further carried out with 250 ml of 33% methanol, and the eluate was concentrated to dryness to obtain concentrate (3). Then, elution was carried out with 250 ml of 66% methanol and the resulting eluate was concentrated to dryness to obtain concentrate (4). Elution was further carried out with 250 ml of 66% methanol and the resulting eluate was concentrated to dryness to obtain concentrate (5). Then, elution was carried out with 250 ml of 100% methanol and the resulting eluate was concentrated to dryness to obtain concentrate (6). After further elution with 250 ml of 100% methanol, elution was carried out with 250 ml of 100% acetone. The resulting eluate was concentrated to dryness to obtain concentrate (8). Then, elution was carried out with 250 ml of 100% acetone and the resulting eluate was concentrated to dryness to obtain concentrate (9).

Example 12

The freeze-dried powder produced in Example 4 (5 g) was suspended in 500 ml of water. The resulting suspension was passed through a column packed with a hydrophobic adsorbent resin (Mitsubishi Chemical Corporation, DIAION HP-20, void volume: 100 ml), followed by elution with 250 ml of 33% methanol. Elution was further carried out with 250 ml of 33% methanol, and the eluate was concentrated to dryness to obtain concentrate (3). Then, elution was carried out with 250 ml of 66% methanol and the resulting eluate was concentrated to dryness to obtain concentrate (4). Elution was further carried out with 250 ml of 66% methanol and the resulting eluate was concentrated to dryness to obtain concentrate (5). Then, elution was carried out with 250 ml of 100% methanol and the resulting eluate was concentrated to dryness to obtain concentrate (6). After further elution with 250 ml of 100% methanol, elution was carried out with 250 ml of 100% acetone. The resulting eluate was concentrated to dryness to obtain concentrate (8). Then, elution was carried out with 250 ml of 100% acetone and the resulting eluate was concentrated to dryness to obtain concentrate (9).

Example 13

The freeze-dried powder produced in Example 2 (5 g) was suspended in 500 ml of water. The resulting suspension was passed through a column packed with a hydrophobic adsorbent resin (Mitsubishi Chemical Corporation, DIAION HP-20, void volume: 100 ml), followed by elution with 250 ml of 33% methanol. Elution was further carried out with 250 ml of 33% methanol, and the eluate was concentrated to dryness to obtain concentrate (3). Then, elution was carried out with 250 ml of 66% methanol and the resulting eluate was concentrated to dryness to obtain concentrate (4). Elution was further carried out with 250 ml of 66% methanol and the resulting eluate was concentrated to dryness to obtain concentrate (5). Then, elution was carried out with 250 ml of 100% methanol and the resulting eluate was concentrated to dryness to obtain concentrate (6). Elution was further carried out with 250 ml of 100% methanol and the resulting eluate was concentrated to dryness to obtain concentrate (7). Subsequently, elution was carried out with 250 ml of 100% acetone, followed by further elution with 250 ml of 100% acetone. The resulting eluate was concentrated to dryness to obtain concentrate (9).

Example 14

The freeze-dried powder produced in Example 5 (5 g) was suspended in 500 ml of water. The resulting suspension was passed through a column packed with a hydrophobic adsorbent resin (Mitsubishi Chemical Corporation, DIAION HP-20, void volume: 100 ml), followed by elution with 250 ml of 33% methanol. Elution was further carried out with 250 ml of 33% methanol, and the eluate was concentrated to dryness to obtain concentrate (3). Then, elution was carried out with 250 ml of 66% methanol and the resulting eluate was concentrated to dryness to obtain concentrate (4). Elution was further carried out with 250 ml of 66% methanol and the resulting eluate was concentrated to dryness to obtain concentrate (5). Then, elution was carried out with 250 ml of 100% methanol and the resulting eluate was concentrated to dryness to obtain concentrate (6). Elution was further carried out with 250 ml of 100% methanol and the resulting eluate was concentrated to dryness to obtain concentrate (7). Subsequently, elution was carried out with 250 ml of 100% acetone, followed by further elution with 250 ml of 100% acetone. The resulting eluate was concentrated to dryness to obtain concentrate (9).

Example 15

The freeze-dried powder of the acetone extract produced in Example 3 (5 g) was suspended in 500 ml of water. The resulting suspension was passed through a column packed with a hydrophobic adsorbent resin (Mitsubishi Chemical Corporation, DIAION HP-20, void volume: 100 ml). The column was washed successively with two 250 ml portions of 33% methanol, two 250 ml portions of 66% methanol and further two 250 ml portions of 66% methanol. After washing with 250 ml of 100% methanol, elution was carried out with 250 ml of 100% acetone and the resulting eluate was concentrated to dryness to obtain concentrate (8).

Example 16 Inhibiting Activity of Extracts of Hydrangeae Dulcis Folium on D-Galactosamine-induced Rat Hepatopathy

Groups of male Wistar white rats ($150 \pm 20$ g, Japan SLC) were kept for at least 3 days under fixed conditions (temperature: $24 \pm 2°C$, humidity: $60 \pm 5\%$, dark and bright interval: 12 hours) for adaptation, and then fed respectively with the feeds produced in Examples 7-10 (test groups) and the feed produced in Comparative Example 1 (control group) for 15 days. On the 14th day, 350 mg/kg of D-galactosamine (dissolved in physiological saline at a concentration of 35 mg/ml, pH 7.1) was intraperitoneally administered to each rat. Twenty-two hours after the administration of D-galactosamine, each of the rats was subjected to laparotomy under anesthesia with Nembutal and blood was sampled.

The thus obtained blood samples were subjected to measurement of blood GPT activity as an indication of liver function in the following manner. The sampled blood was coagulated and separated by centrifugation to obtain a serum. The GPT level in the obtained serum was measured using Transaminase CII-Test Wako (Wako Pure Chemical Industries, Ltd.). The GPT activity of each test group was calculated as the relative value (%) based on the value of control group expressed as 100%. The value is expressed in terms of average value $\pm$ standard error and the statistical test of significance was carried out by T-test.

The results are shown in Table 1.

Table 1

| Feed | GPT activity (%) | Test of significance |
|---|---|---|
| Feed of Example 7 | $49.8 \pm 21.6$ | p=0.0265 |

Table 1 (continued)

| Feed | GPT activity (%) | Test of significance |
|---|---|---|
| Feed of Example 9 | 40.7 ± 9.7 | p=0.0000212 |
| Feed of Example 8 | 30.5 ± 14.6 | p=0.0079 |
| Feed of Example 10 | 14.3 ± 6.9 | p=0.000373 |

When the feeds of Examples 7-10 were administered, the serum GPT activity which is an indication of liver function disorder was as low as 14.3 to 49.8% of that obtained with the feed of Comparative Example 1. This indicates that hepatopathy was inhibited. In the case of the feed of Example 10, the GPT activity was 14.3%, which indicates that the ethanol extract of the residue of a water extract of Hydrangeae Dulcis Folium produced in Example 5 has a strong hepatopathy inhibiting activity.

During 15 days of the feeding, there was no difference among the groups in weight increase, and no abnormality was recognized in appearance or action.

Example 17 Inhibiting Activity of a Fraction of an Acetone Extract of Hydrangeae Dulcis Folium on Acetaminophen-induced Disorder of Primary Cultured Hepatocytes

Hepatocytes of a rat were separated according to the collagenase perfusion method [Seglen, P. O., Methods in Cell Biology, 13, 29 (1976)]. That is, a male SD rat weighing ca. 130 g was subjected to laparotomy under anesthesia, and 400 ml of a preperfusion liquid [a solution prepared by dissolving 9.5 g of Hanks' Balanced Salt Solution (Gibco), 2.38 g of HEPES (Nacalai Tesque, Inc.), 0.19 g of EGTA (Sigma Chemical Co., Ltd.) and 0.35 g of $NaHCO_3$ (Kishida Chemical Co., Ltd.) in 1 l of water, pH 7.2] maintained at 37°C was perfused through a portal vein at a flow rate of 30 ml/minute. Then, 200 ml of a collagenase solution [a solution prepared by dissolving 9.8 g of Hanks' solution Nissui (1) (Nissui Pharmaceutical Co., Ltd.), 2.38 g of HEPES (Nacalai Tesque, Inc.), 0.35 g of $NaHCO_3$ (Kishida Chemical Co., Ltd.), 0.56 g of $CaCl_2$ (Kishida Chemical Co., Ltd.), 0.02 g of trypsin inhibitor (Sigma Chemical Co., Ltd.) and 0.5 g of collagenase (Sigma Chemical Co., Ltd.) in 1 l of water, pH 7.5] maintained at 37°C was perfused. After being digested, the liver was put in a petri dish, and 20 ml of S-MEM medium (Gibco) was added thereto, followed by mincing with a surgical knife. The hepatocytes were dispersed by pipetting with a 10-ml Komagome pipette and then filtered through a gauze patch and a cell filter (Ikemoto Scientific Technology Co., Ltd.) to obtain a hepatocyte dispersion. The obtained hepatocytes contained not only liver parenchymal cells, but also non-parenchymal cells such as endothelial cells, Kupffer cells, Ito cells and star cells, and liver parenchymal cells alone were purified by centrifugation. That is, the obtained hepatocyte dispersion was centrifuged at a low speed (50 x g) under cooling for one minute, and the precipitated liver parenchymal cells were recovered. This operation was repeated three times to separate and recover the liver parenchymal cells of high purity.

The recovered hepatocytes were suspended in the following basal medium at a density of 1.2 x $10^6$ cells/ml, and wells of a matri gel-coated 6-well plate were seeded with 1.4 ml of the resulting suspension, followed by primary culture under the following culture conditions.

The medium was prepared by adding fetal bovine serum (FBS, 10%), penicillin (50 U/ml), streptomycin (50 μg/ml), insulin ($10^{-8}$ M) and dexamethasone ($10^{-6}$ M) to Waymouth's MB752/1 medium (Gibco) (hereinafter sometimes referred to as a basal medium).

Culturing was carried out in a $CO_2$ incubator (5% $CO_2$, 95% air) at 37°C. Four hours after the seeding, the basal medium was removed and the wells were washed with PBS. Then, 0.7 ml of a fresh basal medium was pipetted into each well, followed by further culturing.

Concentrate (8) obtained in Example 15 was dissolved in dimethyl sulfoxide (hereinafter abbreviated as DMSO) in a concentration of 10 mg/ml, and the resulting solution was diluted 50-fold with the above basal medium to prepare a test solution having a concentration of 200 μg/ml.

Twenty-four hours after the seeding of cells, the test solution was added to wells in an amount of 140 μl per well (final concentration: 20 μg/ml), and one hour later, 50 mM acetaminophen (a hepatopathy inducer) dissolved in the medium was added thereto in an amount of 560 μl per well (final concentration: 20 mM) (test group).

Separately, 24 hours after the seeding of cells, a basal medium containing 2% DMSO was added to wells in an amount of 140 μl per well, and one hour later, 50 mM acetaminophen (a hepatopathy inducer) was added thereto in an amount of 560 μl per well (final concentration: 20 mM) (control group 1).

Further, 24 hours after the seeding of cells, a basal medium containing 2% DMSO was added to wells in an amount of 140 μl per well, and one hour later, the medium was added thereto in an amount of 560 μl per well (control group 2).

Forty-eight hours after the addition of acetaminophen or medium, the number of cells of each group was estimated by means of absorbance by MTT [3-(4,5-dimethyl-2-thiazolyl)-2,5-diphenyl-2H-tetrazolium bromide] assay. That is, the basal medium was removed from each well and 1.4 ml of Waymouth's MB752/1 medium containing 10% PBS containing MTT (10 mg/ml) was pippetted therein. After incubation in a $CO_2$ incubator at 37°C for one hour, 4.2 ml of DMSO was added thereto. After vigorous stirring, the absorbance of each group was measured at 570 nm using a microplate reader (Bio Rad, Model 3550). Evaluation was made in duplicate. The hepatocyte disorder inhibiting rate was calculated by the following equation.

[Equation 1]

Hepatocyte disorder inhibiting rate (%) =

[(A-B) / (C-B)] X 100

A: Absorbance of test group
B: Absorbance of control group 1
C: Absorbance of control group 2

The result is shown in Table 2.

Table 2

| Hepatocyte disorder inhibiting rate (%) | 42.2 |
|---|---|

As shown in Table 2, concentrate (8) obtained in Example 15 inhibited the hepatocyte disorder caused by acetaminophen by 42.2%.

<u>Example 18</u> Inhibiting Activity of Fractions of Extracts of Hydrangeae Dulcis Folium on D-Galactosamine-induced Disorder of Primary Cultured Hepatocytes

Each of concentrates (3), (4), (5), (6), (8) and (9) obtained in Examples 11 and 12 and concentrates (3), (4), (5), (6) and (7) obtained in Examples 13 and 14 was dissolved in DMSO in a concentration of 10 mg/ml. Each of the resulting solutions was diluted 50-fold with the basal medium to prepare a diluted solution (concentration: 200 µg/ml). Rat hepatocytes were seeded and primary-cultured in the same manner as in Example 17. Two hours after the seeding of cells, the medium was removed and the wells were washed with PBS. Then, 0.7 ml of a fresh medium was pippetted into each well, and 140 µl of the diluted solution was added thereto (final concentration: 20 µg/ml). Two hours later, 560 µl of 50 mM D-galactosamine (a hepatopathy inducer) dissolved in the medium was added thereto (final concentration: 20 mM) (test group).

Separately, 2 hours after the seeding of cells, a basal medium containing 2% DMSO was added to wells in an amount of 140 µl per well, and 2 hours later, 50 mM D-galactosamine (a hepatopathy inducer) dissolved in the medium was added thereto in an amount of 560 µl per well (final concentration: 20 mM) (control group 1).

Further, 2 hours after the seeding of cells, a basal medium containing 2% DMSO was added to wells in an amount of 140 µl per well, and 2 hours later, the medium was added thereto in an amount of 560 µl per well (control group 2).

Forty-eight hours after the addition of D-galactosamine or medium, the number of cells of each group was estimated by means of absorbance by MTT assay. That is, the basal medium was removed from each well and 1.4 ml of Waymouth's MB752/1 medium containing 10% PBS containing MTT (10 mg/ml) was pippetted therein. After incubation in a $CO_2$ incubator at 37°C for one hour, 4.2 ml of DMSO was added thereto. After vigorous stirring, the absorbance of each group was measured at 570 nm using a microplate reader (Bio Rad, Model 3550). Evaluation was made in duplicate. The hepatocyte disorder inhibiting rate was calculated by the above equation 1.

The results are shown in Table 3.

Table 3

| Fraction of extract of Hydrangeae Dulcis Folium | Hepatocyte disorder inhibiting rate (%) |
|---|---|
| Concentrate (3) of Example 12 | 59.1 |
| Concentrate (4) of Example 12 | 73.0 |

Table 3 (continued)

| Fraction of extract of Hydrangeae Dulcis Folium | Hepatocyte disorder inhibiting rate (%) |
|---|---|
| Concentrate (5) of Example 12 | 67.7 |
| Concentrate (6) of Example 12 | 45.4 |
| Concentrate (8) of Example 12 | 31.8 |
| Concentrate (9) of Example 12 | 44.9 |
| Concentrate (3) of Example 11 | 56.2 |
| Concentrate (4) of Example 11 | 55.8 |
| Concentrate (5) of Example 11 | 55.9 |
| Concentrate (6) of Example 11 | 54.9 |
| Concentrate (8) of Example 11 | 40.2 |
| Concentrate (9) of Example 11 | 47.0 |
| Concentrate (3) of Example 13 | 76.0 |
| Concentrate (4) of Example 13 | 97.4 |
| Concentrate (5) of Example 13 | 66.8 |
| Concentrate (6) of Example 13 | 89.1 |
| Concentrate (7) of Example 13 | 46.1 |
| Concentrate (3) of Example 14 | 65.2 |
| Concentrate (4) of Example 14 | 79.9 |
| Concentrate (5) of Example 14 | 34.0 |
| Concentrate (6) of Example 14 | 45.8 |
| Concentrate (7) of Example 14 | 33.2 |

As shown in Table 3, the fractions of extracts of Hydrangeae Dulcis Folium inhibited the hepatocyte disorder caused by D-galactosamine by 31.8 to 97.4%.

Example 19 Inhibiting Activity of Fractions of Extracts of Hydrangeae Dulcis Folium on D-Galactosamine/TNF-a-induced Disorder of Primary Cultured Hepatocytes

Each of concentrates (9) obtained in Examples 12, 13 and 14 was dissolved in DMSO in a concentration of 10 mg/ml, and the resulting solution was diluted 50-fold with the basal medium to prepare a diluted solution (concentration: 500 µg/ml).

Rat hepatocytes were seeded and primary-cultured in the same manner as in Example 17. Two hours after the seeding of cells, the medium was removed and the wells were washed with PBS. Then, 0.7 ml of a fresh medium was pippetted into each well, and 140 µl of the diluted solution was added thereto (final concentration: 20 µg/ml). Two hours later, a mixture of 2.5 mM D-galactosamine dissolved in the medium and 2.5 ng/ml TNF-$\alpha$ (hereinafter referred to as GT solution) was added thereto in an amount of 560 µl (final concentration: D-galactosamine, 1 mM; TNF-$\alpha$, 1 ng/ml) (test group).

Separately, 2 hours after the seeding of cells, a basal medium containing 2% DMSO was added to wells in an amount of 140 µl per well, and 2 hours later, GT solution (a hepatopathy inducer) was added thereto in an amount of 560 µl per well (final concentration, D-galactosamine, 1 mM; TNF-$\alpha$, 1 ng/ml) (control group 1).

Further, 2 hours after the seeding of cells, a basal medium containing 2% DMSO was added to wells in an amount of 140 µl per well, and 2 hours later, the medium was added thereto in an amount of 560 µl per well (control group 2).

Forty-eight hours after the addition of GT solution or medium, the number of cells of each group was estimated by means of absorbance by MTT assay. That is, the basal medium was removed from each well and 1.4 ml of Waymouth's MB752/1 medium containing 10% PBS containing MTT (10 mg/ml) was pippetted therein. After incubation in a $CO_2$ incubator at 37°C for one hour, 4.2 ml of DMSO was added thereto. After vigorous stirring, the absorbance of each group was measured at 570 nm using a microplate reader (Bio Rad, Model 3550). Evaluation was made in duplicate. The hepatocyte disorder inhibiting rate was calculated by the above equation 1.

The results are shown in Table 4.

Table 4

| Fraction of extract of Hydrangeae Dulcis Folium | Hepatocyte disorder inhibiting rate (%) |
| --- | --- |
| Concentrate (9) of Example 12 | 34.0 |
| Concentrate (9) of Example 13 | 27.4 |
| Concentrate (9) of Example 14 | 17.2 |

As shown in Table 4, the fractions of extracts of Hydrangeae Dulcis Folium inhibited the hepatocyte disorder caused by a mixture of D-galactosamine and TNF-$\alpha$ by 17.2 to 34.0%.

Example 20

A feed having the following composition was prepared by mixing the ingredients.

| CE-2 (Clea Japan, Inc) | 99 wt% |
| --- | --- |
| Powder produced in Example 5 | 1 wt% |

Example 21

A feed having the following composition was prepared by mixing the ingredients.

| CE-2 (Clea Japan, Inc) | 99 wt% |
| --- | --- |
| Powder produced in Example 3 | 1 wt% |

Comparative Example 2

A feed having the following composition was prepared by mixing the ingredients.

| CE-2 (Clea Japan, Inc) | 99 wt% |
| --- | --- |
| Pine-dex #3 (Matsutani Chemical Industry Co., Ltd.) | 1 wt% |

Example 22 Inhibiting Activity of an Ethanol Extract of the Residue of a Water Extract of Hydrangeae Dulcis Folium on Alcohol/LPS-induced Rat Hepatopathy

Groups of female SD white rats (ca. 200 g, Japan SLC) were kept for at least 3 days under fixed conditions (temperature: 24 ± 2°C, humidity: 60 ± 5%, dark and bright interval: 12 hours) for adaptation and then fed respectively with the feed produced in Example 20 (test group) and the feed produced in Comparative Example 2 (control group) for 15 days. During 15 days of the feeding, there was no difference between the two groups in weight increase, and no abnormality was recognized in appearance or action. On the 14th day, 4 g/kg of alcohol [prepared as a 40% (v/v) ethanol solution] was orally administered to the rats of both groups using a sound. Six hours later, 5 mg/kg of a solution prepared by dissolving LPS (derived from E. coli, Sigma Chemical Co., Ltd.) in physiological saline at a concentration of 5 mg/ml was intravenously injected. Twenty-four hours after the injection, each of the rats was subjected to laparotomy under anesthesia with Nembutal and blood was sampled. The test was carried out on the two groups each consisting of 6 rats.

The thus obtained blood samples were subjected to measurement of blood GPT and GOT activities as indications of liver function in the following manner. The sampled blood was coagulated and separated by centrifugation to obtain a serum. The GPT and GOT levels in the obtained serum were measured using Fuji Drychem System 3500 (Fuji Photo Film Co., Ltd.). The GPT and GOT activities of test group were calculated as the relative values based on the values of control group expressed as 100%. The values are expressed in terms of average value ± standard error and the statistical test of significance was carried out by T-test.

The results are shown in Table 5.

Table 5

|  | Relative value based on control group (%) | Test of significance |
|---|---|---|
| GPT activity | $10.8 \pm 1.6$ | p=0.002 |
| GOT activity | $7.7 \pm 1.1$ | p=0.003 |

In the test group to which the extract of Hydrangeae Dulcis Folium was administered, the serum GPT and GOT activities which are indications of liver function disorder were as low as 10.8% and 7.7% of those of the control group. This indicates that hepatopathy was inhibited. By the test of significance, the difference was recognized as significant (p<0.01).

Example 23 Inhibiting Activity of an Acetone Extract of Hydrangeae Dulcis Folium on D-Galactosamine-induced Rat Hepatopathy

Groups of male SD white rats ($150 \pm 20$ g, Japan SLC) were kept for at least 3 days under fixed conditions (temperature: $24 \pm 2°C$, humidity: $60 \pm 5\%$, dark and bright interval: 12 hours) for adaptation, and then fed respectively with the feed produced in Example 21 (test group) and the feed produced in Comparative Example 2 (control group) for 4 days. The rats of both groups were fasted for 18 hours and then 400 mg/kg of D-galactosamine (dissolved in physiological saline at a concentration of 40 mg/ml) was intraperitoneally administered to each rat. Twenty-two hours after the administration of D-galactosamine, each of the rats was subjected to laparotomy under anesthesia with Nembutal and blood was sampled.

The thus obtained blood samples were subjected to measurement of blood GPT activity as an indication of liver function in the following manner. The sampled blood was coagulated and separated by centrifugation to obtain a serum. The GPT level in the obtained serum was measured using Transaminase CII-Test Wako (Wako Pure Chemical Industries, Ltd.). The GPT activity of test group was calculated as the relative value (%) based on the value of control group expressed as 100%. The value is expressed in terms of average value $\pm$ standard error, and the statistical test of significance was carried out by T-test.

The result is shown in Table 6.

Table 6

| Feed | Extract concentration | GPT activity (%) | Test of significance |
|---|---|---|---|
| Example 21 | 1% | $56.4 \pm 9.9$ | p=0.0069 |

When the feed of Example 21 was administered, the serum GPT activity which is an indication of liver function disorder was as low as 56.4% of that obtained with the feed of Comparative Example 2. This indicates that hepatopathy was inhibited.

During the feeding period, there was no difference between the two groups in weight increase, and no abnormality was recognized in appearance or action.

Example 24 Production of a Freeze-dried Powder of a Water Extract of Saxifraga stolonifera Meerb.

Dry powder of Saxifraga stolonifera Meerb. (1 kg, Shihira Shoten) was extracted twice with 10 l of distilled water at room temperature with stirring for one hour. The obtained extract was concentrated and freeze-dried to obtain 150 g of a freeze-dried powder of a water extract of Saxifraga stolonifera Meerb.

Example 25 Production of a Freeze-dried Powder of an Extract of Saxifraga stolonifera Meerb. Extracted with a 60% Aqueous Solution of Ethanol

Dry powder of Saxifraga stolonifera Meerb. (1 kg) was extracted twice with 10 l of a 60% aqueous solution of ethanol at room temperature with stirring for one hour. The obtained extract was concentrated and freeze-dried to obtain 188 g of a freeze-dried powder of an extract of Saxifraga stolonifera Meerb. extracted with a 60% aqueous solution of ethanol.

Example 26 Production of a Freeze-dried Powder of an Acetone Extract of Saxifraga stolonifera Meerb.

Dry powder of Saxifraga stolonifera Meerb. (1 kg) was extracted twice with 10 l of acetone at room temperature with stirring for one hour. The obtained extract was concentrated and freeze-dried to obtain 164 g of a freeze-dried powder of an acetone extract of Saxifraga stolonifera Meerb.

Example 27 Production of a Freeze-dried Powder of a Hot Water Extract of Saxifraga stolonifera Meerb.

Dry powder of Saxifraga stolonifera Meerb. (1 kg) was extracted with 10 l of boiling distilled water for 30 minutes. The obtained extract was concentrated and freeze-dried to obtain 100 g of freeze-dried powder of a hot waster extract of Saxifraga stolonifera Meerb.

Example 28

The freeze-dried powder produced in Example 24 (5 g) was suspended in 500 ml of water. The resulting suspension was passed through a column packed with a hydrophobic adsorbent resin (Mitsubishi Chemical Corporation, DIAION HP-20, void volume: 100 ml), followed by elution with 250 ml of 33% methanol. Elution was further carried out with 250 ml of 33% methanol, and the eluate was concentrated to dryness to obtain concentrate (3). Then, elution was carried out with 250 ml of 66% methanol, and the resulting eluate was concentrated to dryness to obtain concentrate (4). Elution was further carried out with 250 ml of 66% methanol and the resulting eluate was concentrated to dryness to obtain concentrate (5).

Example 29

The freeze-dried powder produced in Example 25 (5 g) was suspended in 500 ml of water. The resulting suspension was passed through a column packed with a hydrophobic adsorbent resin (Mitsubishi Chemical Corporation, DIAION HP-20, void volume: 100 ml), followed by elution with 250 ml of 33% methanol. Elution was further carried out with 250 ml of 33% methanol, and the eluate was concentrated to dryness to obtain concentrate (3). Then, elution was carried out with 250 ml of 66% methanol, and the resulting eluate was concentrated to dryness to obtain concentrate (4). Elution was further carried out with 250 ml of 66% methanol, and the resulting eluate was concentrated to dryness to obtain concentrate (5). Then, elution was carried out with 250 ml of 100% methanol, and the resulting eluate was concentrated to dryness to obtain concentrate (6).

Example 30

The freeze-dried powder produced in Example 26 (5 g) was suspended in 500 ml of water. The resulting suspension was passed through a column packed with a hydrophobic adsorbent resin (Mitsubishi Chemical Corporation, DIAION HP-20, void volume: 100 ml), followed by elution with 250 ml of 33% methanol. The resulting eluate was concentrated to dryness to obtain concentrate (2). After further elution with 250 ml of 33% methanol, elution was carried out with 250 ml of 66% methanol. The resulting eluate was concentrated to dryness to obtain concentrate (4).

Example 31

The freeze-dried powder produced in Example 27 (5 g) was suspended in 500 ml of water. The resulting suspension was passed through a column packed with a hydrophobic adsorbent resin (Mitsubishi Chemical Corporation, DIAION HP-20, void volume: 100 ml), followed by elution with two 250 ml portions of 33% methanol. Then, elution was carried out with 250 ml of 66% methanol and the resulting eluate was concentrated to dryness to obtain concentrate (4). Elution was further carried out with 250 ml of 66% methanol, and the resulting eluate was concentrated to dryness to obtain concentrate (5).

Example 32

A feed having the following composition was prepared by mixing the ingredients.

| | |
|---|---|
| CE-2 (Clea Japan, Inc.) | 99.0 wt% |
| Powder produced in Example 24 | 1.0 wt% |

Example 33

A feed having the following composition was prepared by mixing the ingredients.

| | |
|---|---|
| CE-2 (Clea Japan, Inc.) | 99.0 wt% |
| Powder produced in Example 25 | 1.0 wt% |

Example 34 Inhibiting Activity of Extracts of Saxifraga stolonifera Meerb. [Water Extract (Example 24) and 60% Ethanol Extract (Example 25)] on D-Galactosamine-induced Rat Hepatopathy

Groups of male SD white rats (150 ± 20 g, Japan SLC) were kept for at least 3 days under fixed conditions (temperature: 24 ± 2°C, humidity: 60 ± 5%, dark and bright interval: 12 hours) for adaptation, and then fed respectively with the feeds produced in Examples 32 and 33 (test groups) and the feed produced in Comparative Example 2 (control group) for 3 days. After the rats were fasted for 18 hours, 400 mg/kg of D-galactosamine (dissolved in physiological saline at a concentration of 40 mg/ml) was intraperitoneally administered to each rat. Then, the rats were fed with the above respective feeds for one day, and each of the rats was subjected to laparotomy under anesthesia with Nembutal and blood was sampled.

The thus obtained blood samples were subjected to measurement of blood GPT activity as an indication of liver function in the following manner. The sampled blood was coagulated and separated by centrifugation to obtain a serum. The GPT level in the obtained serum was measured using Transaminase CII-Test Wako (Wako Pure Chemical Industries, Ltd.). The GPT activity of each test group was calculated as the relative value (%) based on the value of control group expressed as 100%. The value is expressed in terms of average value ± standard error and the statistical test of significance was carried out by T-test.

The results are shown in Table 7.

Table 7

| Feed | Extract concentration | GPT activity (%) | Test of significance |
|---|---|---|---|
| Example 32 | 1% | 35.7 ± 6.1 | p=0.0224 |
| Example 33 | 1% | 29.9 ± 8.0 | p=0.0034 |

When the feeds of Examples 32 and 33 were administered, the serum GPT activity which is an indication of liver function disorder was as low as 35.7% and 29.9%, respectively, of that obtained with the feed of Comparative Example 2. This indicates that hepatopathy was inhibited. The difference was recognized as significant by the test of significance.

During the feeding period, there was no difference among the groups in weight increase, and no abnormality was recognized in appearance or action.

Example 35 Inhibiting Activity of Fractions of an Acetone Extract of Saxifraga stolonifera Meerb. on Acetaminophen-induced Disorder of Primary Cultured Hepatocytes

Experiment was carried out in the same manner as in Example 17 using concentrates (2) and (4) obtained in Example 30.

The results are shown in Table 8.

Table 8

| Concentrate of Example 30 | Hepatocyte disorder inhibiting rate (%) |
|---|---|
| (2) | 46.7 |
| (4) | 46.4 |

As shown in Table 8, the fractions of the acetone extract of Saxifraga stolonifera Meerb. inhibited the hepatocyte disorder caused by acetaminophen by 46.7% and 46.4%, respectively.

Example 36 Inhibiting Activity of Fractions of Extracts of Saxifraga stolonifera Meerb. on D-Galactosamine-induced Disorder of Primary Cultured Hepatocytes

Experiment was carried out in the same manner as in Example 18 using the concentrates obtained in Examples 28, 29, 30 and 31.

The results are shown in Table 9.

Table 9

| Concentrate | Hepatocyte disorder inhibiting rate (%) |
|---|---|
| Concentrate (4) of Ex. 31 | 49.1 |
| Concentrate (5) of Ex. 31 | 37.7 |
| Concentrate (3) of Ex. 28 | 83.8 |
| Concentrate (4) of Ex. 28 | 69.0 |
| Concentrate (5) of Ex. 28 | 111.1 |
| Concentrate (3) of Ex. 29 | 36.1 |
| Concentrate (4) of Ex. 29 | 65.1 |
| Concentrate (5) of Ex. 29 | 56.7 |
| Concentrate (6) of Ex. 29 | 66.6 |
| Concentrate (5) of Ex. 30 | 56.0 |
| Concentrate (4) of Ex. 30 | 66.5 |

As shown in Table 9, the fractions of extracts of Saxifraga stolonifera Meerb. inhibited the hepatocyte disorder caused by D-galactosamine by 36.1 to 111.1%.

Example 37 Inhibiting Activity of Fractions of Extracts of Saxifraga stolonifera Meerb. on D-Galactosamine/TNF-$\alpha$-induced Disorder of Primary Cultured Hepatocytes

Experiment was carried out in the same manner as in Example 19 using the concentrates obtained in Examples 28, 29 and 31.

The results are shown in Table 10.

Table 10

| Concentrate | Hepatocyte disorder inhibiting rate (%) |
|---|---|
| Concentrate (4) of Ex. 31 | 30.3 |
| Concentrate (5) of Ex. 31 | 17.0 |
| Concentrate (3) of Ex. 28 | 32.4 |
| Concentrate (4) of Ex. 28 | 53.6 |
| Concentrate (5) of Ex. 28 | 59.7 |
| Concentrate (4) of Ex. 29 | 58.5 |
| Concentrate (5) of Ex. 29 | 90.6 |
| Concentrate (6) of Ex. 29 | 64.8 |

As shown in Table 10, the fractions of extracts of Saxifraga stolonifera Meerb. inhibited the hepatocyte disorder caused by a mixture of D-galactosamine and TNF-$\alpha$ by 17.0 to 90.6%.

Example 38 Production of a Preparation Containing an Ethanol Extract of the Residue of a Water Extract of Hydrangeae Dulcis Folium

A liver function protecting or improving agent having the following composition was produced by mixing the ingredients.

| | |
|---|---|
| Ethanol extract of the residue of a water extract | 49 g |
| of Hydrangeae Dulcis Folium produced in Example 5 Pine-dex #3 | 49 g |
| Iron (III) pyrophosphate (iron source) | 0.1 g |
| Phoscal EFC (calcium source, Nikko Fine Products) | 1 g |
| Vitamin Mix (Merck & Co., Inc.) | 1 g |

Example 39

The liver function protecting or improving agent produced in Example 38 (20 g) was dispersed into 180 ml of water to produce a liver function protecting or improving drink.

Example 40 Production of Cookies Containing an Ethanol Extract of the Residue of a Water Extract of Hydrangeae Dulcis Folium

Cookies (30 pieces) were prepared from the following ingredients according to a conventional method.

| | |
|---|---|
| Soft flour | 100 g |
| Starch | 74 g |
| Water | 14 g |
| Ethanol extract of the residue of a water extract of Hydrangeae Dulcis Folium produced in Example 5 | 30 g |
| Baking powder | 2 Tsp. |
| Salt | 2 Tsp. |
| Egg | 1 |
| Butter | 80 g |
| Milk | 2 Tbsp. |

Example 41 Production of a Feed Containing 1% Freeze-dried Powder of Example 24

A feed having the following composition was produced by mixing the ingredients.

| | |
|---|---|
| Sucrose (Kishida Chemical Co., Ltd.) | 20.0 wt% |
| Corn oil (Nacalai Tesque, Inc.) | 5.0 wt% |
| Choline bitartrate (Tokyo Kasei Kogyo Co., Ltd.) | 0.4 wt% |
| Corn starch (Nippon Starch Chemical Co., Ltd.) | 39.1 wt% |
| AIN-76 vitamin (Oriental Yeast Co., Ltd.) | 1.0 wt% |
| AIN-76 mineral (Oriental Yeast Co., Ltd.) | 3.5 wt% |
| Cellulose (Oriental Yeast Co., Ltd.) | 5.0 wt% |
| Casein (Wako Pure Chemical Industries, Ltd.) | 25.0 wt% |
| Powder produced in Example 24 | 1.0 wt% |

Example 42 Production of a Feed Containing 1% Freeze-dried Powder of Example 25

A feed having the following composition was produced by mixing the ingredients.

| | |
|---|---|
| Sucrose (Kishida Chemical Co., Ltd.) | 20.0 wt% |
| Corn oil (Nacalai Tesque, Inc.) | 5.0 wt% |
| Choline bitartrate (Tokyo Kasei Kogyo Co., Ltd.) | 0.4 wt% |
| Corn starch (Nippon Starch Chemical Co., Ltd.) | 39.1 wt% |

(continued)

| | |
|---|---|
| AIN-76 vitamin (Oriental Yeast Co., Ltd.) | 1.0 wt% |
| AIN-76 mineral (Oriental Yeast Co., Ltd.) | 3.5 wt% |
| Cellulose (Oriental Yeast Co., Ltd.) | 5.0 wt% |
| Casein (Wako Pure Chemical Industries, Ltd.) | 25.0 wt% |
| Powder produced in Example 25 | 1.0 wt% |

## Claims

1. A liver function protecting or improving agent which comprises a plant of the family Saxifragaceae or an extract of the plant as an active ingredient.

2. The liver function protecting or improving agent according to Claim 1, wherein the plant of the family Saxifragaceae belongs to the genus Saxifraga.

3. The liver function protecting or improving agent according to Claim 2, wherein the plant belonging to the genus Saxifraga is Saxifraga stolonifera Meerb.

4. The liver function protecting or improving agent according to Claim 1, wherein the plant of the family Saxifragaceae belongs to the genus Hydrangea.

5. The liver function protecting or improving agent according to Claim 4, wherein the plant belonging to the genus Hydrangea is Hydrangea macrophylla Seringe var. Thunbergii Makino or Hydrangeae Dulcis Folium.

6. The liver function protecting or improving agent according to any of Claims 1 to 5, wherein the extract of the plant of the family Saxifragaceae is an alcoholic extract of the residue of an aqueous medium extract of the plant of the family Saxifragaceae.

7. The liver function protecting or improving agent according to any of Claims 1 to 6, which is administered orally.

8. The liver function protecting or improving agent according to any of Claims 1 to 7, wherein the liver function is a function affected by alcohol.

9. A food and/or drink which comprises a plant of the family Saxifragaceae or an extract of the plant.

10. The food and/or drink according to Claim 9, which is useful for the protection or improvement of liver function.

11. The food and/or drink according to Claim 10, wherein the liver function is a function affected by alcohol.

12. The food and/or drink according to any of Claims 9 to 11, wherein the plant of the family Saxifragaceae belongs to the genus Saxifraga.

13. The food and/or drink according to Claim 12, wherein the plant belonging to the genus Saxifraga is Saxifraga stolonifera Meerb.

14. The food and/or drink according to any of Claims 9 to 11, wherein the plant of the family Saxifragaceae belongs to the genus Hydrangea.

15. The food and/or drink according to Claim 14, wherein the plant belonging to the genus Hydrangea is Hydrangea macrophylla Seringe var. Thunbergii Makino or Hydrangeae Dulcis Folium.

16. The food and/or drink according to any of Claims 9 to 15, wherein the extract of the plant of the family Saxifragaceae is an alcoholic extract of the residue of an aqueous medium extract of the plant of the family Saxifragaceae.

17. A feed which comprises a plant of the family Saxifragaceae or an extract of the plant.

18. The feed according to Claim 17, which is useful for the protection or improvement of liver function.

19. The feed according to Claim 18, wherein the liver function is a function affected by alcohol.

20. The feed according to any of Claims 17 to 19, wherein the plant of the family Saxifragaceae belongs to the genus Saxifraga.

21. The feed according to Claim 20, wherein the plant belonging to the genus Saxifraga is Saxifraga stolonifera Meerb.

22. The feed according to any of Claims 17 to 19, wherein the plant of the family Saxifragaceae belongs to the genus Hydrangea.

23. The feed according to Claim 22, wherein the plant belonging to the genus Hydrangea is Hydrangea macrophylla Seringe var. Thunbergii Makino or Hydrangeae Dulcis Folium.

24. The feed according to any of Claims 17 to 23, wherein the extract of the plant of the family Saxifragaceae is an alcoholic extract of the residue of an aqueous medium extract of the plant of the family Saxifragaceae.

25. An additive for foods and drinks having liver function protecting or improving activity, which comprises a plant of the family Saxifragaceae or an extract of the plant.

26. The additive for foods and drinks according to Claim 25, wherein the liver function is a function affected by alcohol.

27. The additive for foods and drinks according to Claim 25 or 26, wherein the plant of the family Saxifragaceae belongs to the genus Saxifraga.

28. The additive for foods and drinks according to Claim 27, wherein the plant belonging to the genus Saxifraga is Saxifraga stolonifera Meerb.

29. The additive for foods and drinks according to Claim 25 or 26, wherein the plant of the family Saxifragaceae belongs to the genus Hydrangea.

30. The additive for foods and drinks according to Claim 29, wherein the plant belonging to the genus Hydrangea is Hydrangea macrophylla Seringe var. Thunbergii Makino or Hydrangeae Dulcis Folium.

31. The additive for foods and drinks according to any of Claims 25 to 30, wherein the extract of the plant of the family Saxifragaceae is an alcoholic extract of the residue of an aqueous medium extract of the plant of the family Saxifragaceae.

32. A feed additive having liver function protecting or improving activity, which comprises a plant of the family Saxifragaceae or an extract of the plant.

33. The feed additive according to Claim 32, wherein the liver function is a function affected by alcohol.

34. The feed additive according to Claim 32 or 33, wherein the plant of the family Saxifragaceae belongs to the genus Saxifraga.

35. The feed additive according to Claim 34, wherein the plant belonging to the genus Saxifraga is Saxifraga stolonifera Meerb.

36. The feed additive according to Claim 32 or 33, wherein the plant of the family Saxifragaceae belongs to the genus Hydrangea.

37. The feed additive according to Claim 36, wherein the plant belonging to the genus Hydrangea is Hydrangea macrophylla Seringe var. Thunbergii Makino or Hydrangeae Dulcis Folium.

38. The feed additive according to any of Claims 32 to 37, wherein the extract of the plant of the family Saxifragaceae is an alcoholic extract of the residue of an aqueous medium extract of the plant of the family Saxifragaceae.

**39.** A method of screening for liver function protecting or improving agents, which comprises administering an alcohol and then a lipopolysaccharide to an animal to raise the blood GPT or GOT level of the animal, administering a test substance to the animal, and estimating the activity of the test substance to lower the blood GPT or GOT level of the animal.

**40.** The method according to Claim 39, wherein the animal is a mammal.

**41.** The method according to Claim 39 or 40, wherein the lipopolysaccharide is derived from a microorganism belonging to the group of enteric bacteria.

**42.** The method according to any of Claims 39 to 41, wherein the liver function is a function affected by alcohol.

**43.** A method of protecting or improving liver function in an animal, which comprises feeding the animal with the liver function protecting or improving agent according to any of Claims 1 to 8 or the feed according to any of Claims 17 to 24.

**44.** The method according to Claim 43, wherein the animal is selected from the group consisting of livestock, poultry and cultivated fish.

**45.** A food, drink or feed for the protection or improvement of liver function which comprises a plant of the family Saxifragaceae or an extract of the plant as an active ingredient.

**46.** The food, drink or feed according to Claim 45, wherein the plant of the family Saxifragaceae belongs to the genus Saxifraga.

**47.** The food, drink or feed according to Claim 46, wherein the plant belonging to the genus Saxifraga is Saxifraga stolonifera Meerb.

**48.** The food, drink or feed according to Claim 45, wherein the plant of the family Saxifragaceae belongs to the genus Hydrangea.

**49.** The food, drink or feed according to Claim 48, wherein the plant belonging to the genus Hydrangea is Hydrangea macrophylla Seringe var. Thunbergii Makino or Hydrangeae Dulcis Folium.

**50.** The food, drink or feed according to any of Claims 45 to 49, wherein the extract of the plant of the family Saxifragaceae is an alcoholic extract of the residue of an aqueous medium extract of the plant of the family Saxifragaceae.

**51.** The food, drink or feed according to any of Claims 45 to 50, wherein the liver function is a function affected by alcohol.